# EUROPEAN PATENT SPECIFICATION

(11) **EP 0 329 627 B1**
(45) Date of publication and mention of the grant of the patent: **31.08.1994**
(21) Application number: 89830064.5
(22) Date of filing: 17.02.1989
(51) Int. Cl.: C07H 19/10, A61K 31/70

(54) **Process for the production of crystalline forms of the lithium, potassium and magnesium salts of cytidindiphosphocholine, the crystalline forms thus obtained and their pharmaceutical use**
Verfahren zur Produktion von kristallinen Formen von Lithium-, Potassium- und Magnesiumsalzen von Cytidindiphosphocholin, die so erhaltenen kristallinen Formen und ihre pharmazeutische Verwendung
Procédé pour la production de formes cristallines du sel de lithium, de potassium et de magnésium de cytidindiphosphocholine, les formes cristallines ainsi obtenues et leur utilisation pharmaceutique

(30) Priority: 18.02.1988 IT 4764988
(43) Date of publication of application: 23.08.1989
(73) Proprietor: NUOVO CONSORZIO SANITARIO NAZIONALE del Dott. Paolo Malizia & C. - S.A.S., 00136 Roma (IT)
(72) Inventor: Casini, Giovanni, I-00136 Roma RM (IT)
(74) Representative: Bazzichelli, Alfredo

(56) References cited:
- CHEMICAL ABSTRACTS, vol. 109, no. 16, 17th October 1988, page 369, abstract no.134883t, Columbus, Ohio, US; H. QIAN et al.: "Some improvements on purificationtechnique of cytidine-5'-diphosphocholine",
- & YIYAO GONGYE 1988, 19(5), 196-8
- CHEMICAL ABSTRACTS, vol. 81, no. 9, 2nd September 1974, page 419, abstract no.49983j, Columbus, Ohio, US; & JP-A-73 103 524 (AJINOMOTO CO., INC.) 25-12-1973
- CHEMICAL ABSTRACTS, vol. 80, no. 15, 15th April 1974, page 453, abstract no.83533b, Columbus, Ohio, US; & JP-A-74 00 279 (AJINOMOTO CO., INC.) 05-01-1974
- CHEMICAL ABSTRACTS, vol. 78, no. 7, 19th February 1973, page 532, abstract no.43948t, Columbus, Ohio, US; & JP-A-72 19 017 (KOJIN LTD) 19-09-1972
- CHEMICAL ABSTRACTS, vol. 75, no. 8, 23rd August 1971, page 491, abstract no.64172p, Columbus, Ohio, US; K. KIKUGAWA et al.: "Synthesis of a nucleotidecoenzyme, CDP-choline", & CHEM. PHARM. BULL. 1971,19(5), 1011-16

## Description

The present invention relates to a process for the production of crystalline forms of salts of the acid molecule cytidindiphosphocholine (herebelow indicated as cyticholine) having the following general formula 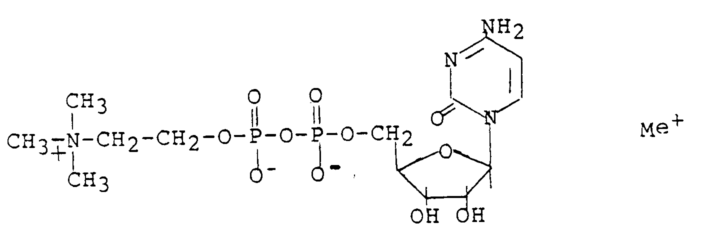
wherein Me⁺ indicates Li⁺, K⁺, or half Mg⁺⁺.

Cyticholine is a normal metabolite present in the nervous tissue of man and of the other higher animals, where it acts as intermedium metabolite in the biosynthesis of phosphatidilcholines (lecithins) essential components of all the cell membranes and in particular of the nervous tissue (Balint et al, J. Lipid Research, 8, 486 (1967)).

Cyticholine also influences indirectly the biosynthesis of other phospholipids, of the ribonucleic acid and of the proteins of the nervous tissue, controls the ATP-asic activity, the mitochondrial respiration, the lipemia and the aggreggation of blood platelets.

Cyticholine has been used for more than ten years, under the form of sodic salt, and in parenteral administration, in the therapy of various traumatic, organic and functional disorders of the central nervous system: aftereffects and disorders of the consciousness as a result of cerebral injuries, of operations on the brain and of serious cerebral alterations, such as cerebral haemorrhage, thrombosis and infarcts; cerebral arteriosclerosis; Parkinson's disease; senile dementia; depressions (J. Am. Med. Assoc. No. 1, January 1986).

In the normally used dosages in the intramuscular and intravenous therapy, up to mg 1000 pro dose, the use of the sodic salt of cyticholine can give rise to disturbances due to electrolytical unbalance, particularly in cardiopathic subjects, in which the heavy introduction of the cation sodium may give rise to a worsening of the pathological pattern.

Cyticholine salts with other cations are not described in the present state of the art; this is due to the fact that the salification with other alkaline or alkaline earth bases leads, after drying, to syrupy or vitreous products, which do not show a crystalline solid form. Also the use of mixtures of solvents leads to the formation of a biphasic liquid system. This makes the application and storage of the product difficult and moreover makes the dosage of the same more difficult.

It has now been surprisingly found that a process comprising a treatment step with acetone of the not completely dried syrup obtained after evaporation of the water solution obtained from the salification of the acid cyticholine with a suitable alkaline or alkaline earth base, makes possible the production of crystalline forms of the lithium, potassium and magnesium salts of cyticholine.

Subject of the present invention is therefore a process for the production of crystalline forms of potassium, lithium and magnesium salts of cytidindiphosphocholine (cyticholine) through reaction in water solution of stoichiometric quantities of cytidindiphosphocholine in acid form, and of an oxide, hydroxide, carbonate or bicarbonate of potassium, lithium or magnesium until, after a non complete evaporation, a non completely dried, syrupy reaction product is obtained, characterized in that:
a) said reaction product is treated with acetone up to the formation of a precipitate; and
b) said formed precipitate is separated through filtration, washed another time with acetone and then vacuum dried.

According to the process of the present invention crystalline forms, well defined from a chemical standpoint, easily water soluble, are obtained, which can usefully substitute the sodic salt of cyticholine showing even advantages due to the specific pharmacological activity of the individual cations used, at the same time allowing optimal storage and dosage of the product obtained.

The crystalline form of the potassic salt of cyticholine can be particularly used in conditions of hypopotassemia, such as the conditions resulting from the use of diuretics, especially if associated to drugs containing digitalis in cardiopathic patients.

The crystalline form of the magnesium salt of cyticholine can be used in view of the sedative action of magnesium when administered parenterally and in view of the choleretic action when administered orally. The crystalline form of the lithium salt of cyticholine can be used for the anti-depressant activity of the lithium cation.

Obviously, all the specific pharmacological activities of the individual cations used join up to the eutrophic action on the nervous system of cyticholine and to its various above referred uses in the pharmaceutical field.

According to the process of the present invention, crystalline forms of alkaline and alkaline earth salts of cyticholine are available which show a pharmacological activity more specific than that of the commonly used sodium salt, said crystalline forms being adaptable to particular pathological conditions in which the sodic salt is not suitable.

### Example of production

### Crystalline form of the potassic salt of cyticholine.

A water solution of acid cyticholine, obtained by the repeated passage of a solution of sodic cyticholine on a cationic exchanger resin, is neutralized with the equivalent quantity of potassium carbonate or bicarbonate; the solution obtained is evaporated at reduced pressure up to a syrupy but not yet vitreous consistency. The syrup thus obtained is slowly treated under agitation with an excess of acetone: a microcrystalline precipitate is formed which is collected on a filter, washed with acetone and dried under vacuum. The yield is about 95%. The crystalline form of the potassium salt of cyticholine is soluble in 0,5 parts of water. It is deliquescent.

### Crystalline form of the magnesium salt of cyticholine

Following the process described for the preparation of the crystalline form of the potassium salt, but using a magnesium oxide, the crystalline form of the magnesium salt of cyticholine is obtained, which is soluble in 1 part of water.

### Crystalline form of the lithium salt of cyticholine

Following the process described for the production of the potassium salt, but this time using lithium hydroxide, the crystalline form of the lithium salt of cyticholine is obtained, which is soluble in 1 part of water.

It is to be pointed out that these salts in solid crystalline form cannot be obtained using any one of the traditional methods of crystalization: neither starting from hydroalcoholic solutions (from which the sodic salt easily crystalizes), nor starting from a mixture of water with other organic solvents, mixable with water. When a fixed concentration of organic solvent is reached clouding and separation of a second liquid phase which is dense and syrupy, are observed. Said phase solidifies neither upon cooling nor upon addition of a primer.

The crystalline forms of the salts of cyticholine produced according to the process of the present invention can be used in injectable preparations in a sterile water solution for parenteral administration (intramuscular or intravenous) or in preparations for oral administration (capsules, pills, solutions) or rectal administration (suppositories).

## Claims

1. A process for the production of crystalline forms of potassium, lithium or magnesium salts of cytidindiphosphocholine through reaction in water solution of stoichiometric quantities of cytidindiphosphocholine, under the form of an acid and of an oxide, hydroxide, carbonate or bicarbonate of potassium, lithium or magnesium until a syrupy reaction product not completely dried is obtained, after a non complete evaporation,
characterized by the fact that:
a) said reaction product is treated by acetone under agitation up to the formation of a precipitate; and
b) said precipitate is separated through filtration, washed with acetone and dried under vacuum.

2. Crystalline form of the potassium salt of cytidindiphosphocholine obtained according to the process as claimed in claim 1.

3. Crystalline form of the lithium salt of cytidindiphosphocholine obtained according to the process as claimed in claim 1.

4. Crystalline form of the magnesium salt of cytidindiphosphocholine obtained according to the process as claimed in claim 1.

5. Use of the crystalline form of the potassium salt of cytidindiphosphocholine as claimed in claim 2, for the production of a drug for the treatment of patients under the necessity of cytidindiphosphocholine and moreover of potassium and unable to tolerate a high dosage of sodium.

6. Use of the crystalline form of the lithium salt of cytidindiphosphocholine as claimed in claim 3, for the production of a drug for the treatment of patients under the necessity of cytidindiphosphocholine and moreover of lithium.

7. Use of the crystalline form of the magnesium salt of cytidindiphosphocholine as claimed in claim 4, for the production of a drug for the treatment of patients under the necessity of cytidindiphosphocholine and moreover of magnesium and unable to tolerate a high dosage of sodium.

## Patentansprüche

1. Verfahren zur Herstellung von kristallinen Formen von Kalium-, Lithium- oder Magnesiumsalzen von Cytidindiphosphocholin durch Reaktion von stöchiometrischen Mengen von Cytidindiphosphocholin in Form einer Säure und eines Oxids, Hydroxids, Carbonats oder Bicarbonats von Kalium, Lithium oder Magnesium in wäßriger Lösung, bis ein dickflüssiges, nicht vollständig getrocknetes Reaktionsprodukt nach einem nicht vollständigen Eindampfprozeß erhalten wird,
gekennzeichnet durch die Tatsache, daß:
a) das Reaktionsprodukt bis zur Bildung eines Niederschlags unter Rühren mit Aceton behandelt wird; und
b) der Niederschlag durch Filtrieren abgetrennt, mit Aceton gewaschen und unter Vakuum getrocknet wird.

2. Kristalline Form des Kaliumsalzes von Cytidindiphosphocholin, erhalten nach dem Verfahren nach Anspruch 1.

3. Kristalline Form des Lithiumsalzes von Cytidindiphosphocholin, erhalten nach dem Verfahren nach Anspruch 1.

4. Kristalline Form des Magnesiumsalzes von Cytidindiphosphocholin, erhalten nach dem Verfahren nach Anspruch 1.

5. Verwendung der kristallinen Form des Kaliumsalzes von Cytidindiphosphocholin nach Anspruch 2 für die Herstellung eines Arzneimittels für die Behandlung von Patienten unter der Notwendigkeit von Cytidindiphosphocholin und darüberhinaus Kalium, die eine hohe Natrium-Dosierung nicht vertragen können.

6. Verwendung der kristallinen Form des Lithiumsalzes von Cytidindiphosphocholin nach Anspruch 3 für die Herstellung eines Arzneimittels für die Behandlung von Patienten unter der Notwendigkeit von Cytidindiphosphocholin und darüberhinaus Lithium.

7. Verwendung der kristallinen Form des Magnesiumsalzes von Cytidindiphosphocholin nach Anspruch 4 für die Herstellung eines Arzneimittels für die Behandlung von Patienten unter der Notwendigkeit von Cytidindiphosphocholin und darüberhinaus Magnesium, die eine hohe Natrium-Dosierung nicht vertragen können.

## Revendications

1. Procédé de production de formes cristallines de sels de potassium, lithium et magnésium de cytidindiphosphocholine par réaction en solution aqueuse de quantités stoechiométriques de cytidindiphosphocholine, sous forme acide, et d'un oxyde, d'un hydroxyde, d'un carbonate ou d'un bicarbonate de potassium, de lithium ou de magnésium, jusqu'à obtention d'un produit de réaction sirupeux incomplètement désséché, après évaporation incomplète, caractérisé :
en ce que :
- a) le dit produit de réaction est traité par l'acétone sous agitation jusqu'à formation d'un précipité, et
- b) le dit précipité est séparé par filtration, lavé à l'acétone et séché sous vide.

2. Forme cristalline du sel de potassium de la cytidindiphosphocholine obtenue selon le procédé de la revendication 1.

3. Forme cristalline du sel de lithium de la cytidindiphosphocholine obtenue selon le procédé de la revendication 1.

4. Forme cristalline du sel de magnésium de la cytidindiphosphocholine obtenue selon le procédé de la revendication 1.

5. Utilisation de la forme cristalline du sel de potassium de la cytidindiphosphocholine selon la revendication 2, pour la production d'un médicament pour le traitement des malades nécéssitant de la cytidindiphosphocholine et de plus du potassium et incapables de supporter une importante dose de sodium.

6. Utilisation de la forme cristalline du sel de lithium de la cytidindiphosphocholine selon la revendication 3, pour la production d'un médicament pour le traitement des malades nécessitant de la cytidindiphosphocholine et de plus du lithium.

7. Utilisation de la forme cristalline du sel de magnésium de la cytidindiphosphocholine selon la revendication 4, pour la production d'un médicament pour le traitement des malades nécessitant de la cytidindiphosphocholine et de plus du magnésium et incapables de supporter une importante dose de sodium.
